# EUROPÄISCHE PATENTSCHRIFT

(11) **EP 1 374 758 B1**
(45) Veröffentlichungstag und Bekanntmachung des Hinweises auf die Patenterteilung: **10.03.2004**
(21) Anmeldenummer: 02405542.8
(22) Anmeldetag: 27.06.2002
(51) Int. Cl.: A61B 3/16

(54) **Vorrichtung zur Erfassung von Messwerten an einem Auge**
Device for detecting measurement data of an eye
Dispositif pour la détection des valeurs de mesure de l'oeil

(43) Veröffentlichungstag der Anmeldung: 02.01.2004
(73) Patentinhaber: SIS AG Surgical Instrument Systems, 2555 Brügg b. Biel (CH)
(72) Erfinder: Rathjen, Dr. Ing. Christian, 28197 Bremen (DE)
(74) Vertreter: BOVARD AG - Patentanwälte

(56) Entgegenhaltungen:
- US-A- 3 272 001
- US-A- 5 088 500
- US-A- 5 355 884
- US-A- 6 135 968
- HILLE K ET AL: "TECHNISCHER AUFBAU, KALIBRIERUNG UND ERGEBNISSE MIT EINEM NEUEN INTRAOKULAREN DRUCKSENSOR MIT TELEMETRISCHER UEBERTRAGUNG CONSTRUCTION, CALIBRATION AND RESULTS OF A NEW SENSOR IN AN INTRAOCULAR LENS WITH TELEMETRIC TRANSMISSION OF THE INTRAOCULAR PRESSURE" KLINISCHE MONATSBLAETTER FUER AUGENHEILKUNDE, FERDINAND ENKE VERLAG, STUTTGART, DE, Bd. 218, Nr. 5, Mai 2001 (2001-05), Seiten 376-386, XP008001250 ISSN: 0023-2165
- SORAB J ET AL: "TACTILE SENSORY MONITORING OF CLINICIAN-APPLIED FORCES DURING DELIVERY OF NEWBORNS" IEEE TRANSACTIONS ON BIOMEDICAL ENGINEERING, IEEE INC. NEW YORK, US, Bd. 35, Nr. 12, 1. Dezember 1988 (1988-12-01), Seiten 1090-1093, XP000004493 ISSN: 0018-9294

## Beschreibung

### Technisches Gebiet

Die vorliegende Erfindung betrifft eine Vorrichtung zur Erfassung von Messwerten an einem Auge. Die Erfindung betrifft insbesondere eine Vorrichtung zur Erfassung von Messwerten an einem menschlichen Auge, welche einen Träger und einen am Träger angebrachten Sensor zur Erfassung der Messwerte am Auge umfasst.

### Stand der Technik

Auf dem Gebiet der Opthalmologie sind verschiedenste Vorrichtungen zur Erfassung von Messwerten an einem menschlichen Auge bekannt. Mit den Fortschritten auf dem Gebiet der Sensortechnik, insbesondere der mikroelektronischen und mikroelektromechanischen Sensortechnik, wurde die Erfassung von Messwerten am menschlichen Auge mittels Sensoren an Stelle von mechanischen Messvorrichtungen möglich. Insbesondere für die Bestimmung des Augeninnendrucks oder des so genannten innerokularen Drucks (IOD) werden vermehrt Augenmessvorrichtungen mit miniaturisierten Druck- oder Kraftsensoren entwickelt und eingesetzt. Selbst mit der Verwendung solcher miniaturisierten Sensoren blieben die bekannten Augenmessvorrichtungen in der Regel allerdings gross dimensioniert, unhandlich und insbesondere nicht für die Selbstapplikation durch Benutzer geeignet.

In der Patentschrift US 3 272 001 wird eine Vorrichtung zum Messen des intraokularen Drucks beschrieben. Die Vorrichtung gemäss US 3 272 001 umfasst eine Messsonde sowie eine Luftpumpe und eine Druckanzeige, die über Luftleitungen mit der Messsonde verbunden sind. Die Messsonde gemäss US 3 272 001 kann mittels eines Rings oder einer Klammer an einem Finger eines Benutzers angebracht werden. Die Messsonde gemäss US 3 272 001 umfasst einen auf das Auge aufsetzbaren mechanischen Stössel, der den intraokularen Druck über die Luftleitungen auf die Druckanzeige überträgt.

### Darstellung der Erfindung

Es ist eine Aufgabe der vorliegenden Erfindung, eine neue Vorrichtung zur Erfassung von Messwerten an einem Auge, insbesondere an einem menschlichen Auge, vorzuschlagen, welche nicht die Nachteile des Stands der Technik aufweist und welche insbesondere leicht zu handhaben und für die Selbstapplikation durch Benutzer geeignet ist.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass in der Vorrichtung zur Erfassung von Messwerten an einem Auge, insbesondere an einem menschlichen Auge, welche einen Träger und einen am Träger angebrachten Sensor zur Erfassung der Messwerte am Auge umfasst, der Träger mit Befestigungsmitteln zur Befestigung der Messvorrichtung an mindestens einem Finger eines Benutzers versehen ist. Die Befestigung des Sensors an einem Finger des Benutzers ermöglicht die Positionierung und Applikation des Sensors mit Hilfe der hohen manuellen motorischen Fähigkeiten des Menschen ohne Zuhilfenahme von komplexen Antrieben, wie zum Beispiel bei den bekannten automatisierten Goldmann Tonometern. Dadurch entfallen auch die möglichen Sicherheitsrisiken der automatisierten Positionierungs- und Messverfahren. Auch die für die Befestigung an einem Finger des Benutzers kompakte und leichte Ausführung der Messvorrichtung reduziert das Verletzungsrisiko. Bei der Applikation des Sensors mittels eines Fingers des Benutzers lassen sich auch Schutzreflexe optimal ausnützen: das Zurückziehen der Hand oder des Fingers ist schneller als das Zurückziehen des Kopfes oder des Oberkörpers. Durch die Befestigung der Messvorrichtung an einem Finger des Benutzers wird insbesondere auch die Selbstapplikation ermöglicht.

Vorzugsweise ist der Träger so ausgestaltet, dass der Sensor im Bereich der Fingerbeere eines Fingers positionierbar ist, wobei der sensitive Bereich des Sensors von der Fingerbeere abgewandt ist. Durch die Positionierung des Sensors im Bereich der Fingerbeere, welche die Fingerspitze und die Fingerkuppe umfasst, kommt der Sensor am Fingerende zu liegen, wo der Mensch sowohl taktil als auch motorisch das für die Applikation grösste Feingefühl und die grösste Geschicklichkeit aufweist. Insbesondere bei der Selbstapplikation wird dadurch eine genaue und feinfühlige Positionierung des Sensors am Auge erreicht. Bei der Positionierung des Sensors im Bereich der Fingerbeere kann durch Aufstützen des Handballens der betreffenden Hand auf der Wange oder dem Kinn und/oder durch Aufstützen des mittleren Fingerglieds des betreffenden Fingers auf dem Jochbein eine äusserst stabile Referenz erzeugt werden, die das Zittern und Wackeln des Sensors und das damit verbundene Verletzungsrisiko stark minimiert und eine einfache und präzise Applikation des Sensors am Auge ohne zusätzliche Positionierungsmittel ermöglicht.

Vorzugsweise ist der Sensor so am Träger angebracht, dass im am Finger befestigten Zustand des Trägers eine bei einer Applikation des Sensors auf das Auge entstehende Anlagekraft auf den Finger übertragbar ist. Dadurch spürt der Benutzer die durch die Applikation des Sensors auf das Auge entstehende Anlagekraft unmittelbar über den Tastsinn, der Benutzer kann bereits erlernte menschliche motorische Fähigkeiten für die Applikation einsetzen und die für die Applikation notwendige Kraft und erforderlichen Bewegungen intuitiv und in ihrer wirklichen Grössenordnung erlernen. Das Erlernen anderer, indirekt vermittelnden Grössen, zum Beispiel der Verschiebeweg eines federgeführten Kontaktkörpers, entfällt.

In einer Ausführungsvariante umfasst die Messvorrichtung Strukturelemente, welche vom sensitiven Bereich des Sensors abgewandt hinter dem Sensor angeordnet sind und welche im am Finger befestigten Zustand des Trägers durch den Benutzer am Finger spürbar sind. Durch solche Strukturelemente können dem Benutzer die genaue Lage des Sensors an seinem Finger und die bei der Applikation des Sensors auf das Auge entstehende Anlagekraft vermittelt werden.

In einer Ausführungsvariante umfasst die Messvorrichtung einen Applikationsring, welcher den Sensor umgibt und welcher bei der Erfassung der Messwerte am Auge anliegt. Ein solcher Applikationsring dient als Applikationshilfe, einerseits indem er bei der Applikation der Messvorrichtung verhindert, dass kantige Ränder des Sensors auf das Auge auftreffen und die Augenoberfläche verletzen, und andererseits indem er als Zentrierhilfe dient.

Vorzugsweise ist im am Finger befestigten Zustand des Trägers die Länge des im Bereich der Fingerbeere liegenden Teils des Trägers auf die Länge des dort gelegenen Endglieds des Fingers beschränkt. Durch die Begrenzung der Ausdehnung des Trägers auf die Länge des Endglieds des Fingers wird die Beweglichkeit des Endglieds, an welchem der Sensor angebracht ist, nicht eingeschränkt und es wird höchste Beweglichkeit des betreffenden Fingers gewährt.

In einer Ausführungsvariante ist der Sensor beweglich am Träger angebracht. Durch eine bewegliche Lagerung des Sensors am Träger wird erreicht, dass sich der Sensor bei einer Applikation mit Kontaktierung des Auges besser an das Auge anlegt und dadurch eine leicht schiefe Applikation korrigiert werden kann.

In einer Ausführungsvariante umfasst die Messvorrichtung Mittel zum Befestigen einer wegwerfbaren Schutzmembran zum Abdecken des Sensors. Die Verwendung einer Schutzmembran über dem Sensor vermindert die Verletzungsgefahr des Auges durch den Sensor. Die durch diese Befestigungsmittel ermöglichte leichte Auswechselbarkeit von Schutzmembranen verringert das Risiko einer Verschmutzung des sensitiven Bereichs des Sensors und erhöht die Hygiene in der Verwendung der Messvorrichtung.

In einer Ausführungsvariante umfasst die Messvorrichtung ein am Träger angebrachtes Schnittstellenmodul zur kontaktbehafteten oder kontaktlosen Datenkommunikation mit einer zur Messvorrichtung externen Auswerteeinheit. Ein solches Schnittstellenmodul ermöglicht die Speicherung, Verarbeitung und Auswertung erfasster Messwerte in einer zur Messvorrichtung externen Einheit, so dass die Grösse und das Gewicht der Messvorrichtung reduziert werden können.

In einer Ausführungsvariante umfasst die Messvorrichtung Verarbeitungsmittel, einen Datenspeicher, eine Anzeige und/oder eine Energiequelle, die am Träger angebracht sind. Dies ermöglicht den selbständigen Einsatz der Messvorrichtung ohne notwendige Verbindung zu zusätzlichen externen Einheiten, was insbesondere bei der Verwendung der Messvorrichtung für die Selbstapplikation bevorzugt ist.

In einer Ausführungsvariante ist der Träger als Bügel ausgestaltet, welcher einen gebogenen Bereich umfasst, der im am Finger befestigten Zustand an der Fingerspitze anliegt. Diese Ausführungsform ist besonders flexibel, da sich der Träger an unterschiedliche Formen und Grössen von Fingerbeeren anpassen kann.

In einer Ausführungsvariante ist der Träger als Fingerhut ausgestaltet. Diese Ausführungsform gewährt einen besonders guten Halt der Messvorrichtung am Finger des Benutzers.

In verschiedenen Ausführungsvarianten umfassen die Befestigungsmittel eine Befestigungsklammer, einen Haftverschluss, ein elastisches Band, einen Ring oder einen Spreizring. Durch verschiedene Ausführungsformen der Befestigungsmittel können unterschiedliche Bevorzugungen der Benutzer berücksichtigt werden. Mittels verstellbaren Befestigungsmitteln wie Spreizringe, elastische Bänder oder Haftverschlüsse, kann die Messvorrichtung auch an verschiedenen Fingern und/oder an mehreren Fingern gleichzeitig befestigt werden.

In verschiedenen Ausführungsvarianten kann der Sensor eben, einen Drucksensor-Array weiter einen Kraftsensor, einen Kontaktsensor, einen Abstandssensor, einen Chemosensor, einen Flächensensor, einen Temperatursensor und/oder ein mikro-optisches Strahler-Empfängermodul umfassen.

In einer Ausführungsvariante umfasst der Sensor eine Lichtquelle als optische Applikationshilfe. Dadurch kann dem Benutzer zusätzlich zum taktilen Feedback ein optisches Signal als Applikationshilfe verfügbar gemacht werden, das dem Benutzer die Distanz des Sensors zum Auge und/oder die Kontaktierung des Sensors mit dem Auge anzeigt.

In einer Ausführungsvariante umfasst die Messvorrichtung einen elektroakustischen Wandler als akustische Applikationshilfe. Dadurch kann dem Benutzer zusätzlich zum taktilen Feedback ein akustisches Signal als Applikationshilfe verfügbar gemacht werden, das dem Benutzer die Annäherung des Sensors an das Auge angibt.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt eine Draufsicht einer Vorrichtung zur Erfassung von Messwerten an einem Auge, welche am Endglied eines Fingers befestigt ist.
Figur 2 zeigt eine Seitenansicht einer Vorrichtung zur Erfassung von Messwerten an einem Auge, welche am Endglied eines Fingers befestigt ist.
Figur 3 zeigt eine räumliche Ansicht einer Vorrichtung zur Erfassung von Messwerten an einem Auge, welche einen als Fingerhut ausgestalteten Träger umfasst.
Figur 3a zeigt eine Seitenansicht einer Vorrichtung zur Erfassung von Messwerten an einem Auge, welche einen als Fingerhut ausgestalteten Träger umfasst, auf welchem ein Sensor angebracht ist.
Figur 3b zeigt eine Seitenansicht einer Vorrichtung zur Erfassung von Messwerten an einem Auge, welche einen als Fingerhut ausgestalteten Träger umfasst, auf welchem ein Sensor in einer alternativen Anordnung angebracht ist.
Figur 3c zeigt eine Seitenansicht einer Vorrichtung zur Erfassung von Messwerten an einem Auge, welche einen als Fingerhut ausgestalteten Träger umfasst, auf dessen Scheitelpunkt ein Sensor angebracht ist.
Figur 4 zeigt eine räumliche Ansicht einer Vorrichtung zur Erfassung von Messwerten an einem Auge, welche einen als Bügel ausgestalteten Träger umfasst, welcher einen gebogenen Bereich umfasst, der im am Finger befestigten Zustand an der Fingerspitze anliegt.
Figur 4a zeigt eine Seitenansicht einer Vorrichtung zur Erfassung von Messwerten an einem Auge, welche einen als Bügel ausgestalteten Träger umfasst, welcher einen gebogenen Bereich umfasst, der im am Finger befestigten Zustand an der Fingerspitze anliegt.
Figur 5a zeigt eine Seitenansicht einer Vorrichtung zur Erfassung von Messwerten an einem Auge, welche einen als Fingerhut ausgestalteten Träger umfasst, auf welchem eine Schutzmembran angebracht ist, die einen am Träger angebrachten Sensor abdeckt.
Figur 5b zeigt eine Schnittansicht eines Träger mit einem darauf angebrachten Sensor, welcher von einem Applikationsring umgeben ist und durch eine am Applikationsring befestigte Schutzmembran abgedeckt ist.
Figur 6 zeigt eine Draufsicht auf den sensitiven Bereich eines Sensors, welcher von einem Applikationsring umgeben ist und ein zentriert angeordnetes Drucksensorelement und mehrere Kontaktsensorsegmente umfasst.
Figur 7 zeigt ein Blockdiagram, welches schematisch ein Elektronikmodul illustriert.

### Wege zur Ausführung der Erfindung

In den Figuren werden einander entsprechende Teile durch gleiche Bezugszeichen bezeichnet.

Das Bezugszeichen 1 bezeichnet eine Vorrichtung zur Erfassung von Messwerten an einem Auge, insbesondere an einem menschlichen Auge. Die Messvorrichtung 1 umfasst einen Träger 11, einen daran angebrachten Sensor 12 und ein mit dem Sensor 12 verbundenes Elektronikmodul 17.

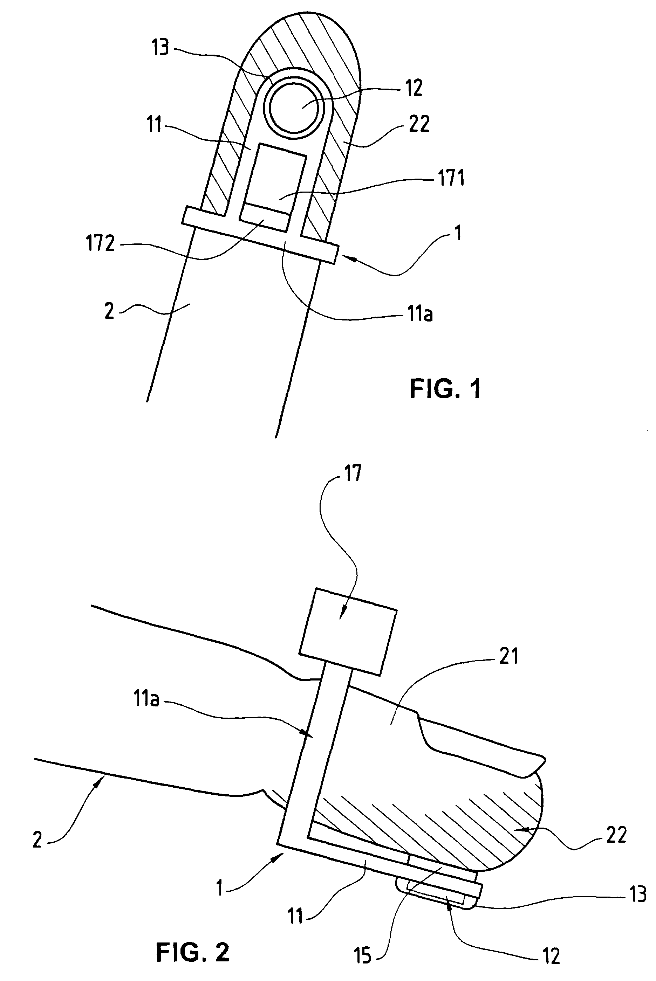

Wie in den Figuren 1 und 2 dargestellt ist, umfasst die Messvorrichtung 1 einen mit dem Träger 11 befestigten Ring 11 a zur Befestigung der Messvorrichtung 1 an einem Finger 2 eines Benutzers, beispielsweise am Zeigefinger. Wie in den Figuren 1 und 2 gezeigt wird, ist der Sensor 12 am freien abgerundeten Ende des zungenförmig ausgestalteten Trägers 11 angebracht und kommt im am Finger 2 befestigten Zustand der Messvorrichtung 1 in den Bereich der Fingerbeere 22 des Fingers 2 zu liegen. Wie in der Figur 2 angedeutet wird, umfasst der Bereich der Fingerbeere 22 die Fingerspitze und die Fingerkuppe des Fingers 2. Der Sensor 12 ist auf der vom Finger 2 abgewandten Seite des Trägers 11 angebracht. Auf der dem Finger 2 zugewandten Seite des Trägers 11, sind hinter dem Sensor 12 Strukturelemente 15 am Träger 11 angebracht. Über die Strukturelemente 15 spürt der Benutzer die Lage des Sensors 12 und die bei der Applikation des Sensors 12 auf das Auge entstehende Anlagekraft über seinen Tastsinn. Die Strukturelemente 15 sind spürbare Erhebungen von geringer Höhe, beispielsweise 0,5 Millimeter. Der sensitive Bereich des Sensors 12 ist vom Träger 11 abgewandt, so dass er durch den Benutzer mit seinem Finger 2 durch draufdrücken oder davorhalten auf ein Auge appliziert werden kann.

Wie in der Figur 2 dargestellt wird, ist der Träger 11 so dimensioniert, dass er sich nicht über die Länge der Endglieds 21 des Fingers 2 hinaus erstreckt, so dass die Beweglichkeit des Endglieds 21 nicht eingeschränkt wird.

Der Sensor 12 ist vorzugsweise beweglich am Träger 11 angebracht, wobei jedoch die bei der Applikation des Sensors 12 auf das Auge entstehende Anlagekraft vom Sensor 12 über den Träger 11 und die Strukturelemente 15 auf die Fingerbeere 22 übertragbar sind. Dem Fachmann sind verschiedene für diesen Zweck geeignete Befestigungsmittel bekannt. Der Sensor 12 kann beispielsweise mittels einer kardanischen Aufhängung am Träger 11 angebracht werden, z.B. als Feinspritzteil aus Plastik (elastokinematisches Gelenk). Der Sensor 12 kann auch durch eine Aufhängung in einer am Träger 11 befestigten Membran 19 am Träger 11 angebracht werden, wie dies z.B. bei Gummitasten von Fernbedienungen gemacht wird. Der Sensor 12 kann auch mittels einer Zugfeder am Träger 11 befestigt und in einer konkaven oder konvexen Kugelpfanne gelagert sein. Eine weitere Möglichkeit besteht darin, den Sensor 12 auf einem am Träger 11 befestigten flexiblen Kissen, z.B. ein Schaumstoff-, Luftoder Silikonkissen, am Träger 11 anzubringen.

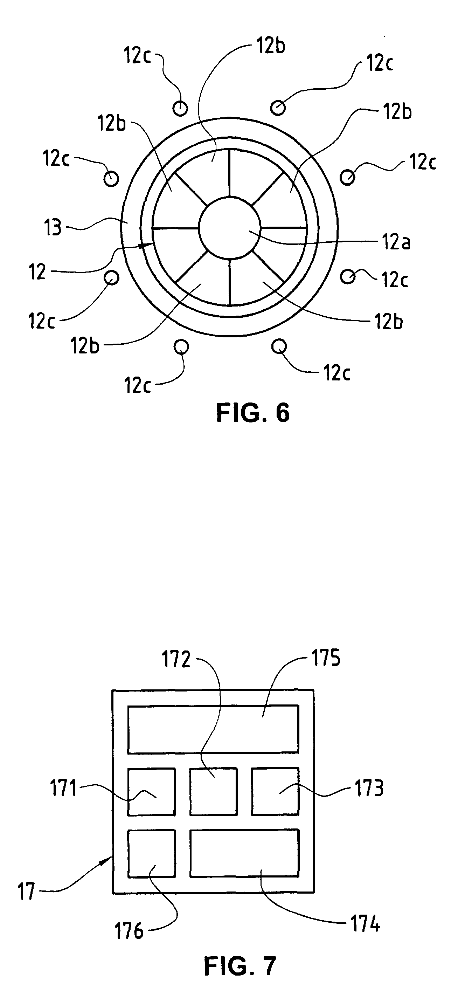

Wie in der Figur 6 illustriert wird, kann der Sensor 12 mehrere Sensorelemente 12a, 12b umfassen. Der in Figur 6 dargestellte sensitive Bereich des Sensors 12 umfasst ein zentriert angeordnetes Drucksensorelement 12a und mehrere Kontaktsensorsegmente 12b, zum Beispiel Streufeldkondensatoren, die konzentrisch um das Drucksensorelement 12a angeordnet sind. Das Drucksensorelement 12a besteht wiederum aus mehreren Drucksensoren, die beispielsweise in einem Array angeordnet sind. Das Drucksensorelement 12a dient zur Bestimmung des Augeninnendrucks. Die Kontaktsensorelemente 12b liefern Kontaktsignale, die zur Applikationshilfe verwendet werden, indem sie ein korrektes Anliegen des Sensors 12 signalisieren. Wie in der Figur 6 schematisch angedeutet wird, sind den einzelnen Kontaktsensorsegmenten 12b jeweils Lichtquellen 12c, beispielsweise LEDs (Light Emitting Diodes), insbesondere mehrfarbige LEDs, geometrisch zugeordnet. Die Lichtquellen 12c werden jeweils abhängig vom Kontaktsignal des ihnen zugeordneten Kontaktsensorsegments 12b angesteuert. Die Lichtquellen 12c sind so angeordnet, dass das von den Lichtquellen 12c ausgestrahlte Licht vom Benutzer direkt von der Lichtquelle 12c und/oder als Reflexion auf der Augenoberfläche gesehen werden kann. In Kombination mit vorzugsweise konzentrisch angeordneten Abstandssensoren können die Lichtquellen 12c zudem als Applikationshilfe zum Einhalten eines bestimmten Abstands zum Auge verwendet werden. Es liesse sich auch eine einzelne zentrale Lichtquelle verwenden. Eine entsprechende Applikationshilfe kann auch durch grafische Wiedergabe der Kontaktsignale, respektive Abstandssignale, auf einer Anzeige erreicht werden.

Neben Drucksensor-Arrays und Kontaktsensoren kann der Sensor 12 auch mit Kräftesensoren, Abstandssensoren, Chemosensoren, Flächensensoren, Temperatursensoren und/oder mikro-optischen Strahler-Empfängermodulen bestückt werden, so dass neben der Bestimmung des Augeninnendrucks auch der Puls, die Körpertemperatur am Augenhintergrund, die Durchblutung des Auges und verschiedene Biomarker der Tränenflüssigkeit, der Retina, der Vorderkammerflüssigkeit, der Hornhaut oder des Schlemmschen Kanals bestimmt werden können, und dass neben der Bestimmung des Augenkontakts zur Applikationshilfe auch der Augenabstand und die Kontaktierungsfläche zur Applikationshilfe bestimmt werden kann. An dieser Stelle soll angefügt werden, dass die Applikation des Sensors 12 zur Bestimmung des Augeninnendrucks die Kontaktierung des Sensors 12 mit dem Auge (insbesondere mit der Hornhaut, dem Lid oder der Sklera) beinhaltet, dass jedoch bei der Bestimmung anderer physiologischer Messwerte, beispielsweise bei der Bestimmung des Pulses oder der Durchblutung, die Applikation des Sensors 12 unter Wahrung einer bestimmten Distanz zum Auge erfolgt.

Obwohl dies nicht dargestellt wurde soll hier erwähnt werden, dass die Messvorrichtung 1 zusätzlich mit Aktuatorelementen versehen werden kann, welche vom sensitiven Bereich des Sensors 12 abgewandt hinter dem Sensor 12 so angeordnet werden, dass sie im am Finger 2 befestigten Zustand der Messvorrichtung 1 durch den Benutzer am Finger 2 spürbar sind. Die Messvorrichtung 1 kann zudem mit einem Treibermodul versehen werden, welche die Aktuatorelemente in Abhängigkeit von den durch den Sensor 12 erfassten Messsignalen ansteuert. Auf diese Weise kann eine aktive Kräfterückkopplung (Force Feedback) zwischen Finger 2 und Sensor 12 zur Verstärkung der empfundenen Anlagekraft beziehungsweise zur aktiven Ausrichtung des Sensors 12 erreicht werden.

Wie in der Figur 7 schematisch dargestellt ist, umfasst das Elektronikmodul 17 Verarbeitungsmittel 171, einen Datenspeicher 172, ein Schnittstellenmodul 173, eine Energiequelle 174 (z.B. eine Batterie), eine Anzeige 175 und einen elektroakustischen Wandler 176. Das Elektronikmodul 17 kann auch einfacher ausgeführt sein. Um die Beweglichkeit des Endglieds nicht zu beeinträchtigen, ist das Elektronikmodul 17 auf der von der Fingerbeere 22 abgewandten Seite angeordnet und, wie in der Figur 2 gezeigt wird, beispielsweise am Ring 11a befestigt. Das Elektronikmodul 17 kann entfernbar mit der Messvorrichtung 1 verbunden sein, so dass es mit verschiedenen Typen von Sensoren 12 elektrisch verbindbar ist, wobei unterschiedliche Sensortypen durch Kenncodes identifizierbar sind, die durch das Elektronikmodul 17 über die entfernbare Verbindung mit der Messvorrichtung 1 detektierbar sind. Wie in der Figur 1 dargestellt wird, können gewisse Teile des Elektronikmoduls 17 auch im Träger 11 ausgeführt sein: die Verarbeitungsmittel 171 und der Datenspeicher 172 können z.B. in CMOS-Technologie (Complementary Metal Oxyd Semi-Conductor) in den Träger 11 integriert werden. In einer Ausführungsvariante sind sowohl die Verarbeitungsmittel 171 und der Datenspeicher 172 als auch der Sensor 12 in einem gemeinsamen CMOS-Chip integriert.

Die Verarbeitungsmittel 171 umfassen Analog-Digital-Wandler zur Umwandlung der durch den Sensor 12 aufgenommenen analogen Messsignale in digitale Messwerte sowie einen Prozessor oder ein Logikmodul. Werden die Messsignale des Sensors 12 auf optischem Wege erzeugt, zum Beispiel über interferometrische Verfahren, können die Messsignale über Lichtleiter zum Elektronikmodul 17 übertragen werden. In diesem Fall umfasst die Messvorrichtung 1 zusätzlich elektrooptische Wandler. Der Datenspeicher 172 dient neben der Speicherung von erfassten Messwerten gegebenenfalls auch zur Speicherung von programmierten Softwaremodulen für die Steuerung des Prozessors. Mittels der Verarbeitungsmittel 171 werden die durch den Sensor 12 aufgenommenen Messsignale verarbeitet, skaliert und der Anzeige 175, dem elektroakustischen Wandler 176 und/oder dem Schnittstellenmodul 173 zugeführt. Das Schnittstellenmodul 173 umfasst Kontakte zur Verdrahtung mit einer zur Messvorrichtung 1 externen Verarbeitungseinheit oder ein Sendermodul für die kontaktlose Datenübertragung an diese externe Verarbeitungseinheit. Die Anzeige 175 dient zur Darstellung von Messwerten und/oder optischen Applikationshilfen. Der elektroakustische Wandler 176 dient zur hörbaren Wiedergabe von Signalen für die Applikationshilfe, beispielsweise unterschiedliche Tonhöhe und/oder Lautstärke in Abhängigkeit von der Distanz des Sensors 12 vom Auge. Die Energiequelle 174 umfasst eine Batterie, eine photovoltaische Solarzelle oder einen Anschluss für eine zur Messvorrichtung 1 externe Speisung.

In einer Ausführungsvariante kann die Anzeige 175 in einem vom Träger 11 separaten Gehäuse angeordnet und mit Befestigungsmitteln zur Befestigung an einem Arm des Benutzers versehen sein, so dass sie vom Benutzer wie eine Armbanduhr getragen werden kann. Die derart angeordnete Anzeige 175 ist für den Datenaustausch kontaktlos oder kontaktbehaftet mit dem Schnittstellenmodul 173 verbunden. Im selben Gehäuse wie die derart angeordnete Anzeige 175 können auch die Verarbeitungsmittel 171, der Datenspeicher 172 und/oder die Energiequelle 174 untergebracht sein.

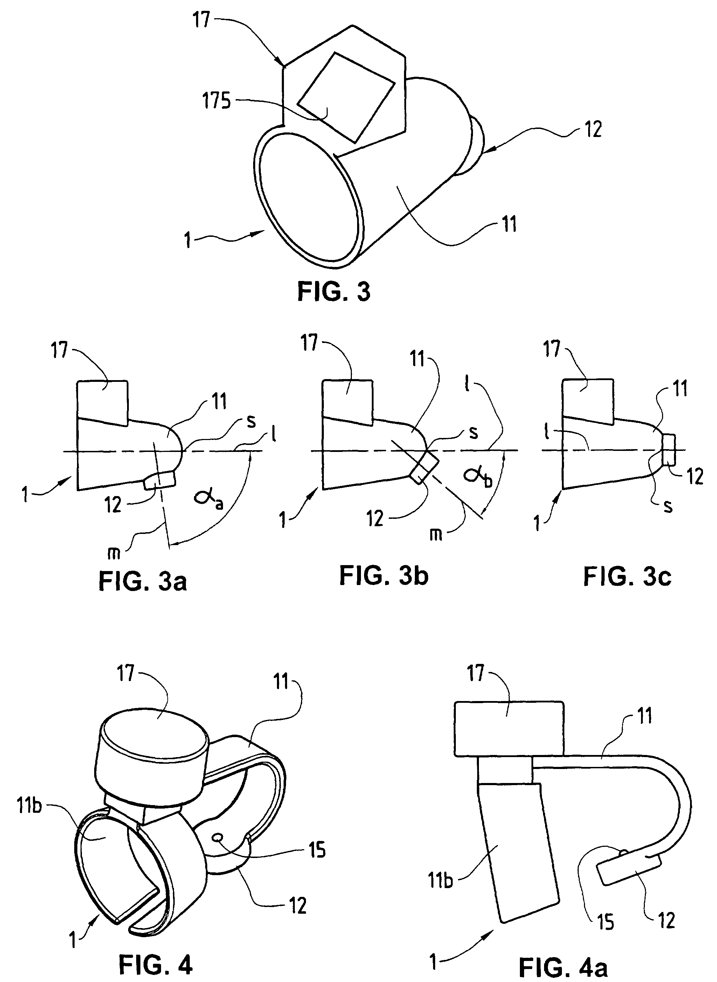

In der Figur 3 wird eine Messvorrichtung 1 zur Erfassung von Messwerten an einem Auge dargestellt, deren Träger 11 als Fingerhut ausgestaltet ist. Der als Fingerhut ausgestaltete Träger 11 kann über das Endglied 21 des Fingers 2 gestülpt werden und kann starr, beispielsweise aus Plastik, oder flexibel, beispielsweise aus Gummi, ausgeführt werden. Die Figur 3 illustriert zudem eine Ausführungsvariante, in der die Anzeige 175 so auf einer abgewinkelten Oberfläche des Elektronikmoduls 17 angeordnet ist, dass sie bei der Applikation des Sensors 12 für eine Zweitperson gut sichtbar ist. Durch Speicherung von Messwerten können die bestimmten Messwerte dem Benutzer jedoch auch nach einer Selbstapplikation auf der Anzeige 175 dargestellt werden.

In den Figuren 3a, 3b und 3c werden verschiedene Ausführungsvarianten der Messvorrichtung 1 dargestellt, in denen der Sensor 12 jeweils unterschiedlich auf dem als Fingerhut ausgestalteten Träger 11 angeordnet ist. In der Ausführungsvariante gemäss den Figuren 3a respektive 3b ist die Mittelachse m des Sensors 12 um den Winkel αₐ respektive α_{b} von der Längsachse I des Trägers 11 geneigt, wobei der Winkel αₐ ungefähr 80° respektive der Winkel α_{b} ungefähr 40° beträgt. In der Ausführungsvariante gemäss der Figur 3c ist der Sensor 12 axial auf dem Scheitelpunkt S des als Fingerhut ausgestalteten Trägers 11 angeordnet. Der als Fingerhut ausgestaltete Träger 11 kann jeweils so am Finger gedreht werden, dass der Sensor 12 im am Finger 2 befestigten Zustand der Messvorrichtung 1 in den Bereich der Fingerbeere 22 des Fingers 2 zu liegen kommt. Die verschiedenen Anordnungen des Sensors 12 gemäss den Figuren 3a, 3b und 3c bedingen jedoch eine unterschiedliche Applikation durch den Benutzer. Die Anordnung gemäss der Figur 3a ermöglicht eine stabilere und bequemere Applikation zentral auf der Hornhaut als die beiden anderen Anordnungen gemäss den Figuren 3b und 3c, da einerseits der Finger 2 in ungekrümmter Lage verwendet werden kann und da andererseits eine bessere Abstützung des Handballens und des Fingers 2 auf der Wange respektive dem Jochbein der zu untersuchenden Person ermöglicht wird. Die Anordnung gemäss der Figur 3b ist beispielsweise besser geeignet, wenn die Applikation auf der Sklera zwischen Nase und Hornhaut erfolgt oder wenn die Messvorrichtung 1 ohne Kontaktierung direkt vor die Hornhaut gehalten wird. Verschiedene Messvorrichtungen 1 mit unterschiedlichen Anordnungen des Sensors 12 gemäss den Figuren 3a, 3b und 3c können durch verschiedene Benutzer mit unterschiedlichen bevorzugten Handhabungen verwendet werden.

Die in den Figuren 3a und 3b dargestellten Anordnungen der Sensoren 12 können auch zu einer Ausführung der Vorrichtung 1 mit zwei verschiedenen Sensoren 12 kombiniert werden, wobei bei der Applikation beispielsweise ein erster Sensor 12 zur Messung des Augeninnendrucks auf dem Auge aufliegt und ein zweiter Sensor 12 zur Messung des Blutsauerstoffwerts sich ohne Kontaktierung vor dem Auge befindet.

In den Figuren 4 und 4a wird eine Vorrichtung 1 zur Erfassung von Messwerten an einem Auge dargestellt, die einen als Bügel ausgestalteten Träger 11 umfasst, welcher einen gebogenen Bereich umfasst, der im am Finger befestigten Zustand an der Fingerspitze anliegt. Der als Bügel ausgestaltete Träger 11 ist an einem Ende mit einem Spreizring 11b verbunden, der über das Endglied 21 des Fingers 2 geschoben wird. Am freien Ende des als Bügel ausgestalteten Trägers 11 ist der Sensor 12 so angebracht, dass er im am Finger 2 befestigten Zustand der Messvorrichtung 1 in den Bereich der Fingerbeere 22 des Fingers 2 zu liegen kommt. Hinter der vom sensitiven Bereich des Sensors abgewandten Rückseite des Sensors 12 sind die oben beschriebenen Strukturelemente 15 angebracht. Der Spreizring 11b ist vorzugsweise entfernbar mit dem Träger 11 verbunden, so dass Spreizringe 11b von unterschiedlicher Grösse zum Anpassen an unterschiedliche Fingergrössen auswechselbar mit dem Träger 11 verbunden werden können.

Verglichen mit der in den Figuren 1 und 2 dargestellten Ausführungsvariante der Messvorrichtung 1 weisen die in den Figuren 3, 3a, 3b, 3c, 4, und 4a dargestellten Ausführungsvarianten der Messvorrichtung 1 eine stabilere Befestigung am Finger 2 auf. Im Unterschied zu der ersteren Ausführungsvariante können allerdings die im Bereich der Fingerspitze liegenden Bereiche der Träger 11 der letzteren Ausführungsvariante bei der Applikation des Sensors 12 am Auge hinderlich werden.

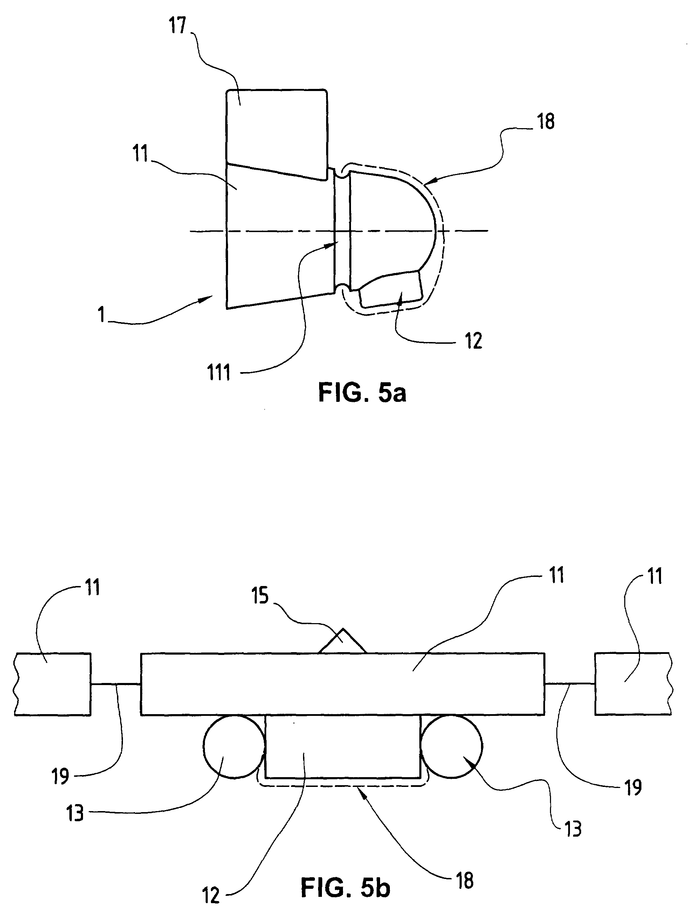

Wie in den Figuren 1, 2 und 5b dargestellt ist, wird der Sensor 12 von einem Applikationsring 13 umgeben, der beispielsweise als flexibler Gummischlauch ausgeführt ist und als Applikationshilfe dient. Wie in der Figur 5b gezeigt wird, kann der Applikationsring 13 auch mit einer Schutzmembran 18 versehen sein, welche den Sensor 12 abdeckt. Die Schutzmembran 18 ist beispielsweise eine dünne und flexible Latex-, Teflon-, Mylar- oder Nylonmembran, welche die Messung nicht beeinflusst. Der mit der Schutzmembran 18 versehene Applikationsring 13 dient als Befestigungsmittel zur Befestigung der Schutzmembran 18 und kann vorzugsweise entfernbar über den Sensor 12 gestülpt werden, so dass er am Sensor 12 anliegt und die Schutzmembran 18 den Sensor 12 abdeckt.

Die Schutzmembran 18 kann zur Abdeckung des Sensors 12 auch formschlüssig mit der Messvorrichtung 1 verbunden werden. Wie in der Figur 5 dargestellt wird, kann der als Fingerhut ausgestaltete Träger 11 beispielsweise mit einer umlaufenden Nut 111 versehen sein, die zur Befestigung einer als dehnbare Schutzkappe ausgestalteten Schutzmembran 18 dient.

Schliesslich kann die Membran 18 wenigstens teilweise aus selbsthaftendem Material gefertigt werden, so dass sie zur Abdeckung des Sensors 12 einfach und entfembar mit der Vorrichtung 1 verbunden werden kann.

In einer Ausführungsvariante ist die Sensoroberfläche (mit dem sensitiven Bereich des Sensors 12) und/oder der Applikationsring 13 konkav ausgeführt, beispielsweise ähnlich einer Kontaktlinse. Bedingt durch die Oberflächenspannung der Tränenflüssigkeit kann sich der Sensor 12 so bei der Applikation durch selbstständiges Ansaugen zentrieren. Bei Verwendung einer genügend elastischen Lagerung des Sensors 12 kann dabei nach erfolgter Applikation durch Rücknahme der Applikationskraft der Einfluss von Störungen durch Fingerbewegungen auf die Messung reduziert werden.

Abschliessend soll angefügt werden, dass der Fachmann neben den dargestellten Befestigungsmitteln den Träger 11 auch mit anderen Mitteln zur Befestigung an einem oder mehreren Fingern des Benutzers versehen kann. Insbesondere verstellbare Befestigungsmittel wie Spreizringe, elastische Bänder oder Haftverschlüsse, wie beispielsweise Klettverschlüsse, ermöglichen die Befestigung der Messvorrichtung 1 an verschiedenen Fingern von unterschiedlicher Grösse und/oder an mehreren Fingern.

## Patentansprüche

1. Vorrichtung (1) zur Erfassung von Messwerten an einem Auge, insbesondere an einem menschlichen Auge, welche einen Träger (11) und einen am Träger (11) angebrachten Sensor (12) zur Erfassung der Messwerte am Auge umfasst, wobei der Träger (11) mit Befestigungsmitteln (11 a, 11 b) zur Befestigung der Vorrichtung (1) an mindestens einem Finger (2) eines Benutzers versehen ist, **dadurch gekennzeichnet,**
**dass** der Sensor (12) einen Drucksensor-Array (12a) umfasst.

2. Vorrichtung (1) nach Anspruch 1, **dadurch gekennzeichnet, dass** der Träger (11) so ausgestaltet ist, dass der Sensor (12) im Bereich der Fingerbeere (22) eines Fingers (2) positionierbar ist, wobei der sensitive Bereich des Sensors (12) von der Fingerbeere (22) abgewandt ist.

3. Vorrichtung (1) nach Anspruch 2, **dadurch gekennzeichnet, dass** der Sensor (12) so am Träger (11) angebracht ist, dass im am Finger (2) befestigten Zustand des Trägers (11) eine bei einer Applikation des Sensors (12) auf das Auge entstehende Anlagekraft auf den Finger (2) übertragbar ist.

4. Vorrichtung (1) nach einem der Ansprüche 2 oder 3, **dadurch gekennzeichnet, dass** die Vorrichtung (1) Strukturelemente (15) umfasst, welche vom sensitiven Bereich des Sensors (12) abgewandt hinter dem Sensor (12) angeordnet sind und welche im am Finger (2) befestigten Zustand des Trägers (11) durch den Benutzer am Finger (2) spürbar sind.

5. Vorrichtung (1) nach einem der Ansprüche 2 bis 4, **dadurch gekennzeichnet, dass** die Vorrichtung (1) einen Applikationsring (13) umfasst, welcher den Sensor (12) umgibt und welcher bei der Erfassung der Messwerte am Auge anliegt.

6. Vorrichtung (1) nach einem der Ansprüche 2 bis 5, **dadurch gekennzeichnet, dass** im am Finger (2) befestigten Zustand des Trägers (11) die Länge des im Bereich der Fingerbeere (22) liegenden Teils des Trägers (11) auf die Länge des dort gelegenen Endglieds (21) des Fingers (2) beschränkt ist.

7. Vorrichtung (1) nach einem der Ansprüche 2 bis 6, **dadurch gekennzeichnet, dass** der Sensor (12) beweglich am Träger (11) angebracht ist.

8. Vorrichtung (1) nach einem der Ansprüche 2 bis 7, **dadurch gekennzeichnet, dass** sie Mittel (111) zum Befestigen einer wegwerfbaren Schutzmembran (18) zum Abdecken des Sensors (12) umfasst.

9. Vorrichtung (1) nach einem der Ansprüche 2 bis 8, **dadurch gekennzeichnet, dass** sie ein am Träger (11) angebrachtes Schnittstellenmodul (173) umfasst zur kontaktbehafteten oder kontaktlosen Datenkommunikation mit einer zur Vorrichtung (1) externen Auswerteeinheit.

10. Vorrichtung (1) nach einem der Ansprüche 2 bis 9, **dadurch gekennzeichnet, dass** sie Verarbeitungsmittel (171), einen Datenspeicher (172), eine Anzeige (175) und/oder eine Energiequelle (174) umfasst, und dass die Verarbeitungsmittel (171), der Datenspeicher (172), die Anzeige (175) und/oder die Energiequelle (174) am Träger (11) angebracht sind.

11. Vorrichtung (1) nach einem der Ansprüche 2 bis 10, **dadurch gekennzeichnet, dass** der Träger (11) als Bügel ausgestaltet ist, welcher einen gebogenen Bereich umfasst, der im am Finger (2) befestigten Zustand an der Fingerspitze anliegt.

12. Vorrichtung (1) nach einem der Ansprüche 2 bis 10, **dadurch gekennzeichnet, dass** der Träger (11) als Fingerhut ausgestaltet ist.

13. Vorrichtung (1) nach einem der Ansprüche 2 bis 11, **dadurch gekennzeichnet, dass** die Befestigungsmittel eine Befestigungsklammer, einen Haftverschluss, ein elastisches Band, einen Ring (11a) oder einen Spreizring (11b) umfassen.

14. Vorrichtung (1) nach einem der Ansprüche 2 bis 13, **dadurch gekennzeichnet, dass** der Sensor (12) weiter einen Kraftsensor, einen Kontaktsensor (12b), einen Abstandssensor, einen Chemosensor, einen Flächensensor, einen Temperatursensor und/oder ein mikro-optisches Strahler-Empfängermodul umfasst.

15. Vorrichtung (1) nach einem der Ansprüche 2 bis 14, **dadurch gekennzeichnet, dass** der Sensor (12) eine Lichtquelle (12c) als optische Applikationshilfe umfasst.

16. Vorrichtung (1) nach einem der Ansprüche 2 bis 15, **dadurch gekennzeichnet, dass** sie einen elektroakustischen Wandler (176) als akustische Applikationshilfe umfasst.

## Claims

1. Device (1) for detecting measurement data of an eye, in particular a human eye, which device comprises a support (11) and a sensor (12) fixed to the support (11) for detecting measurement data on the eye, the support (11) being provided with fastening means (11a, 11b) for fastening the device (1) to at least one finger (2) of a user, **characterised**
**in that** the sensor (12) comprises a pressure sensor array (12a).

2. Device (1) according to claim 1, **characterised in that** the support (11) is designed such that the sensor (12) is positionable in the region of the fingerpad (22) of a finger (2), the sensitive region of the sensor (12) being remote from the fingerpad (22).

3. Device (1) according to claim 2, **characterised in that** the sensor (12) is fixed to the support (11) in such a way that in the state of the support (11) fastened to the finger (2) a pressing force arising during an application of the sensor (12) to the eye is transmittable to the finger (2).

4. Device (1) according to one of the claims 2 or 3, **characterised in that** the device (1) comprises structural elements (15) which are disposed behind the sensor (12) and remote from the sensitive region of the sensor (12), and which are perceivable on the finger (2) by the user in the state of the support (11) fastened to the finger (2).

5. Device (1) according to one of the claims 2 to 4, **characterised in that** the device (1) comprises an application ring (13) which encircles the sensor (12) and which abuts the eye during the detection of the measurement data.

6. Device (1) according to one of the claims 2 to 5, **characterised in that**, in the state of the support (11) fastened to the finger (2), the length of the part of the support (11) situated in the region of the fingerpad (22) is limited to the length of the distal phalanx (21), situated there, of the finger (2).

7. Device (1) according to one of the claims 2 to 6, **characterised in that** the sensor (12) is movably mounted on the support (11).

8. Device (1) according to one of the claims 2 to 7, **characterised in that** it comprises means (111) for attaching a disposable protective membrane (18) for covering the sensor (12).

9. Device (1) according to one of the claims 2 to 8, **characterised in that** it comprises an interface module (173) fixed to the support (11) for data communication, with contacts or contactless, with an evaluation unit external to the device (1).

10. Device (1) according to one of the claims 2 to 9, **characterised in that** it comprises processing means (171), a data store (172), a display (175) and/or an energy source (174), and **in that** the processing means (171), the data store (172), the display (175) and/or the energy source (174) are fixed to the support (11).

11. Device (1) according to one of the claims 2 to 10, **characterised in that** the support (11) is designed as a bow comprising a curved area which rests on the finger tip in the state fastened to the finger (2).

12. Device (1) according of one of the claims 2 to 10, **characterised in that** the support (11) is designed as a thimble.

13. Device (1) according to one of the claims 2 to 11, **characterised in that** the fastening means comprise a fastening clamp, an adhesive closure, an elastic band, a ring (11a) or a spreader ring (11b).

14. Device (1) according to one of the claims 2 to 13, **characterised in that** the sensor (12) further comprises a force sensor, a contact sensor (12b), a distance sensor, a chemosensor, a surface sensor, a temperature sensor and/or a micro-optical emitter-receiver module.

15. Device (1) according to one of the claims 2 to 14, **characterised in that** the sensor (12) comprises a light source (12c) as an optical application aid.

16. Device (1) according to one of the claims 2 to 15, **characterised in that** it comprises an electro-acoustical converter (176) as an acoustical application aid.

## Revendications

1. Dispositif (1) pour la détection de valeurs de mesure d'un oeil, en particulier sur un oeil humain, dispositif qui comprend un support (11) et un capteur (12) placé sur le support (11) pour la détection des valeurs de mesure sur l'oeil, le support (11) étant muni de moyens de fixation (11a, 11b) pour la fixation du dispositif (1) sur au moins un doigt d'un utilisateur, **caractérisé en ce que**
le capteur (12) comprend un réseau de capteurs de pression (12a).

2. Dispositif (1) selon la revendication 1, **caractérisé en ce que** le support (11) est réalisé de sorte que le capteur (12) est positionnable dans la zone de la pulpe digitale (22) d'un doigt (2), la zone sensible du capteur (12) étant détournée de la pulpe digitale (22).

3. Dispositif (1) selon la revendication 2, **caractérisé en ce que** le capteur (12) est disposé sur le support (11) de sorte que quand le support (11) est fixé sur le doigt (2), une force d'application se développant lors d'une application du capteur (12) sur l'oeil, peut être transmise au doigt (2).

4. Dispositif (1) selon l'une des revendications 2 ou 3, **caractérisé en ce que** le dispositif (1) comprend des éléments de structure (15) qui sont disposés derrière le capteur (15) en étant détournés de la zone sensible du capteur (12) et qui sont détectables par l'utilisateur sur le doigt (2) à l'état fixé du support (11) sur le doigt (2).

5. Dispositif (1) selon l'une des revendications 2 à 4, **caractérisé en ce que** le dispositif (1) comprend une bague d'application (13) qui entoure le capteur (12) et qui s'applique contre l'oeil lors de la détection des valeurs de mesure.

6. Dispositif (1) selon l'une des revendications 2 à 5, **caractérisé en ce que** dans l'état du support (11) fixé sur le doigt (2), la longueur de la partie du support (11) se trouvant dans la zone de la pulpe digitale (22) est limitée à la longueur de la phalange d'extrémité (21) du doigt (2).

7. Dispositif (1) selon l'une des revendications 2 à 6, **caractérisé en ce que** le capteur (12) est placé mobile sur le support (11).

8. Dispositif (1) selon l'une des revendications 2 à 7, **caractérisé en ce qu'**il comprend des moyens (111) pour la fixation d'une membrane de protection jetable (18) pour le recouvrement du capteur (12).

9. Dispositif (1) selon l'une des revendications 2 à 8, **caractérisé en ce qu'**il comprend un module d'interface (173) disposé sur le support (11) pour la communication de données avec ou sans contact avec une unité d'évaluation externe au dispositif (1).

10. Dispositif (1) selon l'une des revendications 2 à 8, **caractérisé en ce qu'**il comprend des moyens de traitement (171), une mémoire de donnée (172), un écran (175) et/ou une source d'énergie (174) et **en ce que** les moyens de traitement (171), la mémoire de données (172), l'affichage (175) et/ou la source d'énergie (174) sont placés sur le support (11).

11. Dispositif (1) selon l'une des revendications 2 à 10, **caractérisé en ce que** le support (11) est réalisé comme un étrier qui comprend une zone coudée qui s'appuie sur la pointe du doigt à l'état fixé sur le doigt (2).

12. Dispositif (1) selon l'une des revendications 2 à 10, **caractérisé en ce que** le support (11) est réalisé comme un dé à coudre.

13. Dispositif (1) selon l'une des revendications 2 à 11, **caractérisé en ce que** les moyens de fixation comprennent une agrafe de fixation, une fermeture adhésive, une bande élastique, une bague (11a) ou une bague d'écartement (11b).

14. Dispositif (1) selon l'une des revendications 2 à 13, **caractérisé en ce que** le capteur (12) comprend en outre un capteur de force, un capteur de contact (12b), un capteur de distance, un capteur chimique, un capteur de surface, un capteur thermique et/ou un module émetteur-récepteur micro-optique.

15. Dispositif (1) selon l'une des revendications 2 à 14, **caractérisé en ce que** le capteur (12) comprend une source de lumière (12c) en tant qu'auxiliaire d'application optique.

16. Dispositif (1) selon l'une des revendications 2 à 15, **caractérisé en ce qu'**il comprend un convertisseur électro-acoustique (176) en tant qu'auxiliaire d'application optique.
